# EUROPEAN PATENT SPECIFICATION

(11) **EP 3 483 258 B1**
(45) Date of publication and mention of the grant of the patent: **13.01.2021**
(21) Application number: 17893308.1
(22) Date of filing: 14.08.2017
(51) Int. Cl.: C12N 1/20, C12P 7/24, C12R 1/11

(54) **METHOD FOR PRODUCING P-HYDROXYBENZALDEHYDE USING MICROORGANISM**
VERFAHREN ZUR HERSTELLUNG VON P-HYDROXYBENZALDEHYD UNTER VERWENDUNG EINES MIKROORGANISMUS
PROCÉDÉ DE PRODUCTION DE P-HYDROXYBENZALDÉHYDE METTANT EN OEUVRE UN MICRO-ORGANISME

(30) Priority: 20.01.2017 CN 201710046221
(43) Date of publication of application: 15.05.2019
(73) Proprietor: Xiamen Oamic Biotechnology Co., Ltd., Xiamen City, Fujian 361000 (CN)
(72) Inventor: ZHAO, Xijing, Xiamen Fujian 361000 (CN); XING, Chenguang, Xiamen Fujian 361000 (CN); HUANG, Zhiqiang, Xiamen Fujian 361000 (CN); MA, Fang, Xiamen Fujian 361000 (CN); GONG, Hongcan, Xiamen Fujian 361000 (CN)
(74) Representative: Verscht, Thomas Kurt Albert
(86) International application number: PCT/CN2017/097297
(87) International publication number: WO 2018/133399

(56) References cited:
- WO-A2-01/92539
- CN-A- 101 074 421
- CN-A- 106 011 002
- JP-A- H06 277 078
- EI-SHISHTAWY: 'Solid fermentation of wheat bran for hydrolytic en- zymes production and saccharification content by a local isolate Bacillus megatherium' BMC BIOTECHNOLOGY vol. 29, no. 1, 24 April 2014, page 29, XP021181800
- FITZPATRICK: 'Characterization of YqjM, an Old Yellow Enzyme Ho log from Bacillus subtilis Involved in the Oxidative Stress Response' THE JOURNAL OF BIOLOGICAL CHEMISTRY vol. 278, no. 22, 26 March 2003, pages 19891 - 19897, XP002439308

## Description

### Technical Field

The invention relates to the field of biotechnology, in particular to a production method of p-hydroxybenzaldehyde by a microorganism.

### Background of the Invention

P-hydroxybenzaldehyde, having a molecular formula of C₇H₆O₂ and a molecular weight of 122.12, is a light yellow or off-white crystal, with a slightly aromatic odor. It is mainly used as important intermediates in the pharmaceutical and fragrance industries.

### 1. Physical properties

P- hydroxybenzaldehyde is a light yellow or off-white crystal with a slightly aromatic odor, melting point being 112∼116 °C, relative density being 1.129 and boiling point being 246.6 °C.

P- hydroxybenzaldehyde is highly soluble in ethanol, ether, acetone, ethyl acetate and DMF, slightly soluble in water (solubility is 1.388 g/100 mL in water at 30.5 °C), soluble in benzene (solubility is 3.68 g/mL in benzene at 65 °C).

### 2. Application and Production

P-hydroxybenzaldehyde is an important intermediate in the pharmaceutical and perfume industries. It can be used in the pharmaceutical industry to synthesize amoxicillin, trimethoprim (TMP), 3,4,5-trimethoxybenzaldehyde, p-hydroxyglycine, cefadroxil, artificial gastrodia, farrerol, esmolol, etc. It is used in the fragrance industry for the synthesis of vanillin, ethyl vanillin, piperonal, springaldehyde, 4-anisaldehyde, raspberry ketone and other compounds.

P-hydroxybenzaldehyde can be produced by Pseudomonas putida and Enterobacter species by fermentation (Japanese patent application JP H06 277078).

Bacillus megaterium is a known bacterial species (Korean patent applications CN 106 011 002 A and CN 101 074 421), but so far no strains capable of producing p-hydroxybenzaldehyde have been described.

### Summary of the Invention

The main purpose of the present invention is to provide a strain of *Bacillus* megaterium OMK-11 with p-hydroxybenzaldehyde production capacity. The strain has been deposited at the China Center for Type Culture Collection (CCTCC) with No. M 2016658. The place of deposit is Wuhan University, Wuhan City, The date of deposit is November 20, 2016.

Another purpose of the invention is to provide the application of the said strain in the production of p-hydroxybenzaldehyde.

The further purpose of the present invention is to provide a production method of p-hydroxybenzaldehyde, which adopts the aforementioned strain.

The invention provides a bacterial strain with p-hydroxybenzaldehyde production capacity and uses the strain to produce p-hydroxybenzaldehyde via fermentation. The fermentation method produces p-hydroxybenzaldehyde by using natural raw materials such as saccharides, amino acids, etc., and generates the target products through microbial metabolism. The method is a production method with low temperature and low pressure, relatively safe, simple operation and less pollution, and is an environmentally friendly production method. The invention has a higher output and is a production method with industrialization prospect.

### Brief Description of the Drawings

Fig. 1 is a liquid chromatogram of L- tyrosine standard in embodiment 1.
Fig. 2 is a liquid chromatogram of p-hydroxybenzaldehyde standard in embodiment 1.
Fig. 3 is a liquid chromatogram of p-hydroxybenzaldehyde fermentation liquid in embodiment 1.

### Detailed Description

### The strain

*Bacillus* megaterium OMK-11, a strain with p-hydroxybenzaldehyde production capacity, was isolated from the soil of ginger plantation. The strain was deposited at the China Center for Type Culture Collection on November 20, 2016. The deposit number is CCTCC NO: M 2016658.

The strain can utilize glucose, sucrose, ribose and arabinose. The 18S rRNA and 26S rRNA sequences of the strain are 99% similar to the sequences of known Bacillus standard strains. The strain is identified as Bacillus, with rod-shaped, rounded at the end, single or short chain arrangement and Gram-positive.

The activation of the strain, the cultivation of the seed and the fermentation can be carried out in the following ways:
1) the activation of the strain : under sterile conditions, a full inoculation loop of liquid taken from the glycerol stock is uniformly spread on the solid slant medium, and cultured in a biochemical incubator at 20-37 °C for 24-48 hours;
2) the cultivation of the seed:
   under sterile conditions, a well-grown culture is taken from the solid slant medium with a sterile inoculation loop and used to inoculate the sterile seed culture medium, the initial pH of the seed culture medium is 5-8, and the strain is cultured to the logarithmic growth phase at the temperature of 28-35 °C and the rotation speed of 150-500 rpm;
   the seed culture medium is, calculated by the mass ratio, formulated as follows: 1.0-3.5% sugar raw materials, 0.1-1.0% inorganic salts and 0.1-1.0% yeast extract powder;
   3) the fermentation: the seed liquid cultured to the logarithmic growth phase is introduced to the fermentation medium at a volume ratio of 5-20% under sterile conditions;
   the initial pH of the fermentation medium is 3.0-10.0, at the temperature of 30-40 °C and the rotation speed of 200-500 rpm, under ventilation conditions, the inoculated fermentation medium is fermented for 7-20 h, after which the ventilation is reduced and the fermentation medium is continued to be fermented to 70-80 h;
   the fermentation medium, by mass ratio, consists of 2.0-5.0% sugar raw materials, 0.1-1.0% inorganic salts, 1.0-2.5% yeast extract powder and 1.0-3.0% tyrosine.

The solid slant medium is preferably a broth medium.

The said sugar raw materials include at least one of glucose, sucrose and starch.

The inorganic salt includes a potassium salt or a sodium salt.

The initial pH of the fermentation medium of step 3) is preferably 5.0-9.0.

### Embodiment 1

### The cultivation of the seed

Under sterile conditions, a well-grown strain culture from a solid slant medium was used to inoculate a sterile seed culture medium. The initial pH of the seed culture medium was 5, and the strain were cultured to the logarithmic growth phase at the temperature of 28 °C and the rotation speed of 200 rpm.

The seed culture medium was formulated by mass ratio as follows: 1.0% glucose, 0.5% NaCl and 0.1% Yeast extract powder.

### The fermentation process

The seed liquid cultured to the logarithmic growth phase was used to inoculate the fermentation medium at a volume ratio of 10% under sterile conditions. The initial pH of the fermentation medium was 5.0. The inoculated fermentation medium was fermented for 15 h at 40 °C, at the rotation speed of 200 rpm and the ventilation rate of 1:0.1. After 15 h, the ventilation was reduced to 1:0.01 and the fermentation medium was continued to be fermented to 55 h.

The fermentation was formulated as follows: 1% glucose, 0.5% NaCl, 1% yeast extract powder, 2% tyrosine and trace elements.

Analysis of fermentation liquid: high performance liquid chromatography (HPLC).
Column type: COSMOSIL;5C₁₈-AR-II;4.6ID*250MM.
Column temperature: 30 °C.
Mobile phase: the ratio of methanol to water (0.1% phosphoric acid) is 55:45
Current Speed: 0.8 mL/min,UV: 216 nm.

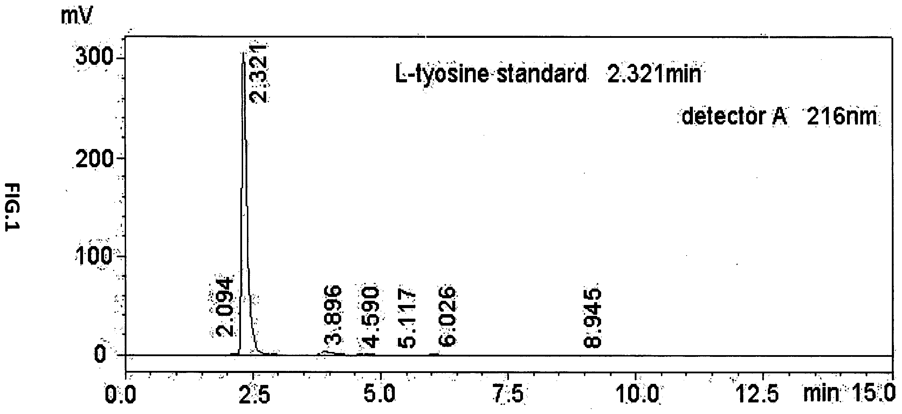

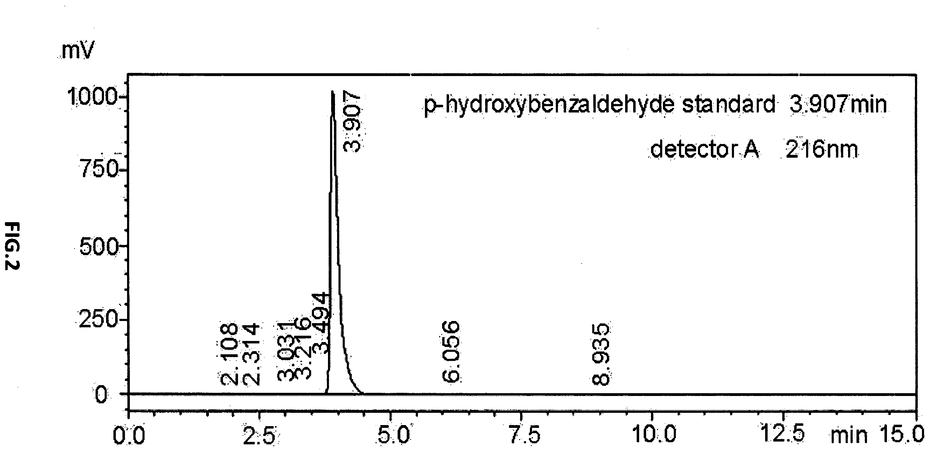

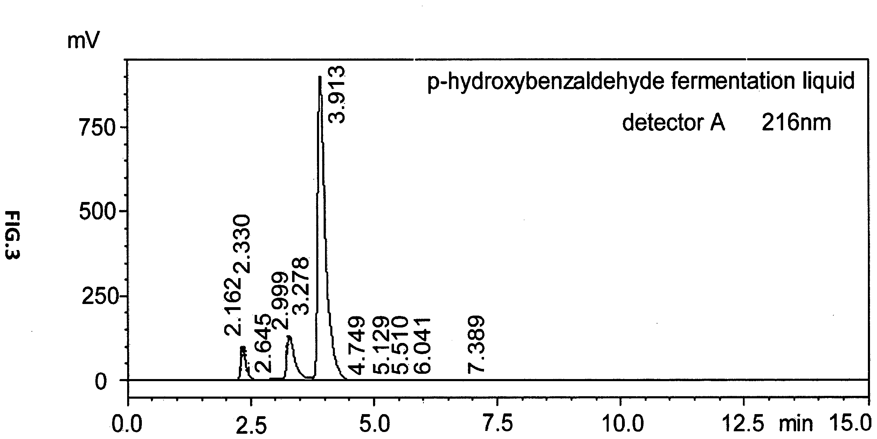

The results are shown in Figures 1 to 3. The results show that the fermentation liquid contains p-hydroxybenzaldehyde.

### Embodiment 2

### The cultivation of the seed

Under sterile conditions, from the solid slant medium, a well-grown culture was taken with a sterile inoculation loop and introduced into the sterile seed culture medium. The initial pH of the seed culture medium was 6, and the strain was cultured to the logarithmic growth phase at the temperature of 30 °C and the rotation speed of 250 rpm.

The seed culture medium was formulated as follows: 3.5% sucrose, 0.5% KCI and 1 % Yeast extract powder.

### The fermentation

The seed liquid cultured to the logarithmic growth phase was added to the fermentation medium at a volume ratio of 10% under sterile conditions. The initial pH of the fermentation medium was 9.0. The inoculated fermentation medium was fermented for 7 hours at 35 °C, 350 rpm rotation speed and 1:0.2 ventilation rate. After 7 h, the ventilation was reduced to 1:0.02 and the fermentation medium was continued to be fermented to 73 h.

The fermentation formula was composed of 4% sucrose, 0.5% KCI, 1.0-2.5% yeast extract powder, 1.0-3.0% tyrosine and trace elements.

### Embodiment 3

### The cultivation of the seed

Under sterile conditions, from the solid slant medium, a well-grown culture was taken with a sterile inoculation loop and used to inoculate the sterile seed culture medium. The initial pH of the seed culture medium was 8, and the strain was cultured to the logarithmic growth phase at the temperature of 35 °C and the rotation speed of 150 rpm.

The seed culture medium was formulated as follows: 3.5% starch, 0.5% NaCl and 1 % Yeast extract powder.

### The fermentation

The seed liquid cultured to the logarithmic growth phase, was used to inoculate the fermentation medium at a 15% volume ratio under sterile conditions. The initial pH of the fermentation medium was 7.0, and the inoculated fermentation medium was fermented for 10 h at 35 °C, 400 rpm rotation speed and 1:0.1 ventilation rate. After 10 h, the ventilation was reduced to 1:0.01 and the fermentation medium was continued to be fermented to 65 h. The fermentation medium was formulated as follows:

4% starch, 1.0% KCl, 2.5% yeast extract powder, 3% tyrosine and trace elements.

### Embodiment 4

The production of natural p-hydroxybenzaldehyde (oscillating fermentation of triangular bottles)

The seed culture medium was prepared as follows : 10g/L glucose, 7g/L potassium dihydrogen phosphate, 2.5g/L sodium chloride and 10g/L yeast extract powder. The solvent used was water, and the initial pH was 7. The seed culture medium was sterilized at 121 °C for 30 min.

The fermentation medium was prepared as follows: 20 g/L glucose, 4 g/L potassium hydrogen phosphate, 1.0 g/L yeast extract powder, 10.0 g/L tyrosine and 100 microliter trace element. The initial pH was 7.5, and the solvent was water. The fermentation medium was sterilized at 121 °C for 30 min.

The preparation of the seed: under sterile conditions, the glycerol stock of the OMK-11 strain, stored at low temperature, was transferred to a fresh, sterile solid medium plate and the inoculated solid media plate was incubated at 28 °C for 2 days. A single colony was picked and used to inoculate a 500 mL flask for the seed culture. The volume of the seed culture medium in the bottle was 50 mL. The flask was rotated at 180 rpm and cultured at 30 °C for 24 hours to obtain the seed liquid.

The fermentation of natural p-hydroxybenzaldehyde: The 50 mL fermentation medium was poured into a 500 mL sterile triangular flask, and then 7.5 mL seed liquid was added for fermentation. The fermentation temperature was 30 °C and the rotating speed of the shaker was 200 rpm. After shaking the fermentation flask for 20 h, the rotation speed of shaker was reduced to 80 rpm, and the fermentation medium was continued to be fermented to 80 h. Finally, the concentration of natural p-hydroxybenzaldehyde in the fermentation medium was determined by HPLC ,the concentration was 2.0g/L.

Embodiment 5 The production of natural p-hydroxybenzaldehyde (bioreactor fermentation)

The seed culture medium and the fermentation medium were prepared in the same manner as embodiment 4.

The preparation of the seed: under sterile conditions, the glycerol stock of the OMK-11 strain, stored at low temperature, was transferred to a fresh, sterile solid plate and incubated at 28 °C for 2 days. A single colony was picked and used to inoculate a 3-liter stainless steel tank containing 1.8 L seed culture medium for the seed culture. The shaker was rotated at 300 rpm and the inoculated seed culture medium is cultured at 30 °C for 24 hours to obtain the seed liquid.

The fermentation of natural p-hydroxybenzaldehyde. 10.2 L of the fermentation medium was added into a 20 L fermenter, which was sterilized at 121 °C for 30 min, and then 1.8 L of the seed liquid was added into the 20 L fermenter for fermentation. The fermentation temperature was 30 °C, and the rotation speed was 400 rpm. The ventilation ratio was 1:0.1, and the inoculated fermentation medium was fermented for 6 h. After 6 h, the ventilation was reduced to 1:0.01, and the fermentation medium was continued to be fermented to 54 h. At the end of fermentation, the concentration of natural p-hydroxybenzaldehyde in the fermentation medium was determined by HPLC method. The concentration was 3 g/L.

### Industrial Applicability

The invention provides a strain with p-hydroxybenzaldehyde production capacity and uses the strain to ferment and produce p-hydroxybenzaldehyde.

## Claims

1. *Bacillus* megaterium OMK-11, a strain with p-hydroxybenzaldehyde production capacity, deposited at the China Center for Type Culture Collection (CCTCC) with No. M 2016658

2. The application of the strain according to claim 1 in the production of p-hydroxybenzaldehyde.

3. A production method of p-hydroxybenzaldehyde which adopts the strain according to claim 1.

4. The production method of p-hydroxybenzaldehyde according to claim 3, wherein, comprises the following steps:
1) the activation of the strain;
2) the cultivation of the seed: in a seed culture medium, the strain is cultured to logarithmic growth phase; the seed culture medium has an initial pH of 5-8, and the seed culture medium by mass ratio is composed of 1.0-3.5% sugar raw materials, 0.1-1.0% inorganic salts and 0.1-1.0% yeast extract powder;
3) the fermentation: a fermentation medium has an initial pH of 3.0-10.0, and the fermentation medium by mass ratio contains 2.0-5.0% sugar raw materials, 0.1-1.0% inorganic salts, 1.0-2.5% yeast extract powder and 1.0-3.0% tyrosine.

5. The production method of p-hydroxybenzaldehyde according to claim 4, wherein:
1) the activation of the strain: under sterile conditions, a full inoculation loop of liquid, which carries the strain from its glycerol stock , is uniformly spread on a solid slant medium, and after spreading, the medium is cultured in a biochemical incubator at 20-37 °C for 24-48 hours;
2) the cultivation of the seed: the inoculated seed culture medium is incubated at 28-35 °C and a rotation speed of 150-500 rpm to the logarithmic growth phase;
3) the fermentation steps comprise: the seed liquid cultured to the logarithmic growth phase is added to the fermentation medium, at a volume ratio of 5-20% under sterile conditions; the fermentation medium is fermented at 30-40 °C and a rotation speed of 200-500 rpm, under ventilation conditions for 7-20 h; after which, the ventilation is reduced and the fermentation medium is continued to be fermented to 50-100 h.

6. The production method of p-hydroxybenzaldehyde according to claim 5, wherein, the solid slant medium mentioned in step 1) is a broth medium.

7. The production method of p-hydroxybenzaldehyde according to claim 4 or 5, wherein, the sugary raw material mentioned in step 2) and 3) comprises at least one of glucose, sucrose, and starch.

8. The production method of p-hydroxybenzaldehyde according to claim 4 or 5, wherein, the inorganic salt mentioned in step 2) and 3) comprises a potassium salt or a sodium salt.

9. The production method of p-hydroxybenzaldehyde according to claim 4 or 5, wherein, the initial pH of the fermentation medium in step 3) is 5.0-9.0.

## Patentansprüche

1. Bacillus megaterium OMK-11, ein Stamm mit p-Hydroxybenzaldehyd-Produktionsfähigkeit, welcher im China Center for Type Culture Collection (CCTCC) mit Nr. M 2016658 hinterlegt ist.

2. Die Anwendung des Stammes nach Anspruch 1 bei der Herstellung von p-Hydroxybenzaldehyd.

3. Ein Herstellungsverfahren von p-Hydroxybenzaldehyd, welches den Stamm nach Anspruch 1 annimmt.

4. Das Herstellungsverfahren für p-Hydroxybenzaldehyd nach Anspruch 3, wobei es die folgenden Schritte aufweist:
1) die Aktivierung des Stammes;
2) die Kultivierung des Keims: in einem Keimkulturmedium wird der Stamm bis zur logarithmischen Wachstumsphase kultiviert; das Keimkalturmedium hat einen anfänglichen pH von 5-8, und das Keimkulturmedium ist nach Massenverhältnis aus 1,0-3,5% Zuckerrohstoffen, 0,1-1,0% anorganischen Salzen und 0,1-1,0% Hefeextraktpulver zusammengesetzt;
3) die Fermentation: ein Fermentationsmedium hat einen anfanglichen pH von 3,0-10,0, und das Fermentationsmedium enthält nach Massenverhältnis 2,0-5,0% Zuckerrohstoffe, 0,1-1,0% anorganische Salze, 1,0-2,5% Hefeextraktpulver und 1,0-3,0% Tyrosin.

5. Das Herstellungsverfahren für p-Hydroxybenzaldehyd nach Anspruch 4, wobei:
1) die Aktivierung des Stammes: unter sterilen Bedingungen wird eine volle Impföse von Flüssigkeit, welche den Stamm von seinem Glycerinvorrat trägt, gleichmäßig auf einem festen schrägen Medium verteilt, und nach Verteilung wird das Medium in einem biochemischen Inkubator bei 20-37°C für 24-48 Stunden kultiviert;
2) die Kultivierung des Keims: Das geimpfte Keimkultunnedium wird bei 28-35°C und einer Rotationsgeschwindigkeit von 150-500 UpM bis zur logarithmischen Wachstumsphase inkubiert;
3) Die Fermentationsschritte weisen auf: die zur logarithmischen Wachstumsphase kultivierte Keimflüssigkeit wird dem Fermentationsmedium hinzugefügt, und zwar in einem Volumenverhältnis von 5-20% unter sterilen Bedingungen; das Fermentationsmedium wird bei 30-40°C und einer Rotationsgeschwindigkeit von 200-500 UpM, unter Belüftungsbedingungen für 7-20 Stunden, fermentiert; danach wird die Belüftung reduziert und das Fermentationsmedium wird bis 50-100 Stunden weiter fermenttiert.

6. Das Herstellungsverfahren für p-Hydroxybenzaldehyd nach Anspruch 5, wobei das in Schritt 1) erwähnte feste schräge Medium ein Brühenmedium ist.

7. Das Herstellungsverfahren für p-Hydroxybenzaldehyd nach Anspruch 4 oder 5, wobei der in Schritt 2) und 3) erwähnte Zuckerrohstoff wenigstens eines von Glucose, Saccharose und Stärke aufweist.

8. Das Herstellungsverfahren für p-Hydroxybenzaldehyd nach Anspruch 4 oder 5, wobei das in Schritt 2) und 3) erwähnte anorganische Salz ein Kaliumsalz oder ein Natriumsalz aufweist.

9. Das Herstellungsverfahren für p-Hydroxybenzaldehyd nach Anspruch 4 oder 5, wobei der anfängliche pH des Fermentationsmediums in Schritt 3) 5,0-9,0 ist.

## Revendications

1. Souche de Bacillus megaterium OMK-11 ayant une capacité de production de p-hydroxybenzaldéhyde, déposée au China Center for Type Culture Collection (CCTCC) avec le numéro M 2016658.

2. Application de la souche de la revendication 1 dans la production de p-hydroxybenzaldéhyde.

3. Procédé de production de p-hydroxybenzaldéhyde qui adopte la souche de la revendication 1.

4. Procédé de production de p-hydroxybenzaldéhyde selon la revendication 3, qui comprend les étapes suivantes :
1) activation de la souche ;
2) culture du germe : dans un milieu de culture de germe, la souche est cultivée jusqu'à la phase de croissance logarithmique ; le milieu de culture de germe a un pH initial de 5 à 8, et le milieu de culture de germe est composé, en proportions en masse, de 1,0 à 3,5 % de sucres matières premières, de 0,1 à 1,0 % de sels inorganiques, et de 0,1 à 1,0 % d'extrait de levure en poudre ;
3) fermentation : un milieu de fermentation a un pH initial de 3,0 à 10,0, et le milieu de fermentation contient, en proportions en masse, 2,0 à 5,0 % de sucres matières premières, 0,1 à 1,0 % de sels inorganiques, 1,0 à 2,5 % d'extrait de levure en poudre et 1,0 à 3,0 % de tyrosine.

5. Procédé de production de p-hydroxybenzaldéhyde selon la revendication 4, dans lequel :
1) activation de la souche : dans des conditions stériles, une boucle d'inoculation complète de liquide, qui transporte la souche depuis son stock de glycérol, est uniformément étalée sur un milieu incliné solide et, après étalement, le milieu est cultivé dans un incubateur biochimique à 20-37°C pendant 24 à 48 heures ;
2) culture du germe : le milieu de culture de germe inoculé est incubé à 28-35°C et à une vitesse de rotation de 150 à 500 t/min jusqu'à la phase de croissance logarithmique ;
3) les étapes de fermentation comprennent : le liquide de germe cultivé jusqu'à la phase de croissance logarithmique est ajouté au milieu de fermentation, en une proportion en volume de 5 à 20 %, dans des conditions stériles ; le milieu de fermentation est fermenté à 30-40°C et à une vitesse de rotation de 200 à 500 t/min, dans des conditions de ventilation pendant 7 à 20 heures ; après quoi la ventilation est réduite et le milieu de fermentation continue d'être fermenté jusqu'à 50-100 heures.

6. Procédé de production de p-hydroxybenzaldéhyde selon la revendication 5, dans lequel le milieu incliné solide mentionné dans l'étape 1) est un milieu de bouillon.

7. Procédé de production de p-hydroxybenzaldéhyde selon la revendication 4 ou 5, dans lequel le sucre matière première mentionné dans les étapes 2) et 3) comprend au moins l'un parmi le glucose, le saccharose, et l'amidon.

8. Procédé de production de p-hydroxybenzaldéhyde selon la revendication 4 ou 5, dans lequel le sel inorganique mentionné dans les étapes 2) et 3) comprend un sel de potassium ou un sel de sodium.

9. Procédé de production de p-hydroxybenzaldéhyde selon la revendication 4 ou 5, dans lequel le pH initial du milieu de fermentation dans l'étape 3) est de 5,0 à 9,0.
